# EUROPEAN PATENT SPECIFICATION

(11) **EP 3 122 746 B1**
(45) Date of publication and mention of the grant of the patent: **25.04.2018**
(21) Application number: 15717812.0
(22) Date of filing: 20.03.2015
(51) Int. Cl.: C07D 417/14, A01N 43/78, A01P 3/00

(54) **PHENYLPIPERIDINECARBOXAMIDE DERIVATIVES AS FUNGICIDES**
PHENYLPIPERIDINECARBOXAMIDE DERIVATE ALS FUNGIZIDE
DÉRIVÉS DE PHENYLPIPERIDINECARBOXAMIDES COMME FONGICIDES

(30) Priority: 24.03.2014 EP 14161339
(43) Date of publication of application: 01.02.2017
(73) Proprietor: Bayer CropScience Aktiengesellschaft, 40789 Monheim am Rhein (DE)
(72) Inventor: HILLEBRAND, Stefan, 41462 Neuss (DE); WASNAIRE, Pierre, 40225 Düsseldorf (DE); WACHENDORFF-NEUMANN, Ulrike, 56566 Neuwied (DE); TSUCHIYA, Tomoki, F-69009 Lyon (FR)
(74) Representative: BIP Patents
(86) International application number: PCT/EP2015/055872
(87) International publication number: WO 2015/144571

(56) References cited:
- WO-A1-2012/055837

## Description

The invention relates to Phenylpiperidinecarboxamide derivatives, to agrochemically active salts thereof, to use thereof and to methods and compositions for controlling phytopathogenic harmful fungi in and/or on plants or in and/or on seed of plants, to processes for producing such compositions and treated seed, and to use thereof for controlling phytopathogenic harmful fungi in agriculture, horticulture and forestry, in animal health, in the protection of materials and in the domestic and hygiene sector. The present invention further relates to a process for preparing Phenylpiperidinecarboxamide derivatives.

It is already known that particular heterocyclically substituted thiazoles can be used as fungicidal crop protection compositions (see WO 07/014290, WO 08/013925, WO 08/013622, WO 08/091594, WO 08/091580, WO 09/055514, WO 09/094407, WO 09/094445, WO 09/132785, WO 10/037479, WO 10/065579, WO 11/076510, WO 11/018415, WO 11/018401, WO 11/076699, WO 11/146182, WO 12/055837, WO 12/025557, WO 12/082580). However, specifically at relatively low application rates, the fungicidal efficacy of these compounds is not always adequate.

Since the ecological and economical demands made on modern crop protection agents are increasing constantly, for example with respect to activity spectrum, toxicity, selectivity, application rate, formation of residues and favourable manufacture, and there can furthermore be problems, for example, with resistances, there is a constant need to develop novel crop protection compositions, in particular fungicides, which, at least in some areas, have advantages over the known ones.

It has now been found that, surprisingly, the present Phenylpiperidinecarboxamide derivatives achieve at least some aspects of the objects mentioned and are suitable for use as crop protection compositions, especially as fungicides.

The invention provides compounds of the formula **(I)** 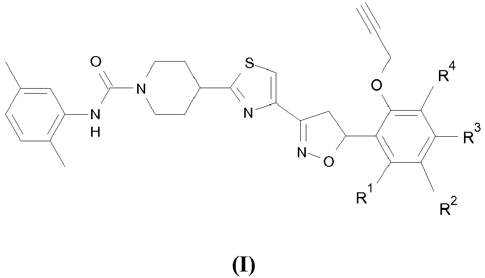 in which the radicals are defined as follows:
- R¹, R², R³ and R⁴: are independently of each other H or fluorine, wherein at least one of these substituents is a fluorine atom.

*Preferred* are compounds of the formula (I) in which the radicals are defined as follows (Table 1):

**Table 1:**

| | **R¹** | **R²** | **R³** | **R⁴** |
|---|---|---|---|---|
| **I-1** | fluoro | hydrogen | hydrogen | fluoro |
| **I-2** | fluoro | hydrogen | hydrogen | hydrogen |
| **I-3** | fluoro | fluoro | hydrogen | hydrogen |
| **I-4** | fluoro | hydrogen | fluoro | hydrogen |

### Elucidation of the preparation processes and intermediates

The Phenylpiperidinecarboxamide derivatives of the formula **(I)** and their intermediates can be prepared in different ways. Either they are prepared in analogy to routes described in the literature for other thiazolylpiperidines, for example WO 12/055837, or as shown schematically below. Unless stated otherwise, the radicals are each as defined above.

The processes according to the invention for preparing compounds of the formula (**I**) are optionally performed using one or more reaction auxiliaries.

Useful reaction auxiliaries are, if required, inorganic or organic bases or acid acceptors. These preferably include alkali metal or alkaline earth metal acetates, amides, carbonates, hydrogencarbonates, hydrides, hydroxides or alkoxides, for example sodium acetate, potassium acetate or calcium acetate, lithium amide, sodium amide, potassium amide or calcium amide, sodium carbonate, potassium carbonate or calcium carbonate, sodium hydrogencarbonate, potassium hydrogencarbonate or calcium hydrogencarbonate, lithium hydride, sodium hydride, potassium hydride or calcium hydride, lithium hydroxide, sodium hydroxide, potassium hydroxide or calcium hydroxide, sodium methoxide, ethoxide, n- or i-propoxide, n-, i-, s- or t-butoxide or potassium methoxide, ethoxide, n- or i-propoxide, n-, i-, s- or t-butoxide; and also basic organic nitrogen compounds, for example trimethylamine, triethylamine, tripropylamine, tributylamine, ethyldiisopropylamine, N,N-dimethylcyclohexylamine, dicyclohexylamine, ethyldicyclohexylamine, N,N-dimethylaniline, N,N-dimethylbenzylamine, pyridine, 2-methyl-, 3-methyl-, 4-methyl-, 2,4-dimethyl-, 2,6-dimethyl-, 3,4-dimethyl- and 3,5-dimethylpyridine, 5-ethyl-2-methylpyridine, 4-dimethylaminopyridine, N-methylpiperidine, 1,4-diazabicyclo[2.2.2]octane (DABCO), 1,5-diazabicyclo[4.3.0]non-5-ene (DBN) or 1,8-diazabicyclo[5.4.0]undec-7-ene (DBU).

The processes according to the invention are optionally performed using one or more diluents. Useful diluents are virtually all inert organic solvents. These preferably include aliphatic and aromatic, optionally halogenated hydrocarbons, such as pentane, hexane, heptane, cyclohexane, methyl cyclohexane, petroleum ether, benzine, ligroin, benzene, toluene, xylene, methylene chloride, ethylene chloride, chloroform, carbon tetrachloride, chlorobenzene and o-dichlorobenzene, ethers such as diethyl ether, methyl tert-butyl ether and dibutyl ether, glycol dimethyl ether and diglycol dimethyl ether, methyltetrahydrofuran, tetrahydrofuran and dioxane, ketones such as acetone, methyl ethyl ketone, methyl isopropyl ketone and methyl isobutyl ketone, esters, such as methyl acetate, ethyl acetate and butyl acetate, nitriles, for example acetonitrile, propionitrile and butyronitrile, alcohols, for example methanol, ethanol, propanol, iso-propanol, butanol, tert-butanol, amides, for example dimethylformamide, dimethylacetamide and N-methylpyrrolidone, and also dimethyl sulphoxide, tetramethylenesulphone and hexamethylphosphoramide and DMPU.

In the processes according to the invention, the reaction temperatures can be varied within a relatively wide range. In general, the temperatures employed are between 0°C and 250°C, preferably temperatures between 10°C and 185°C.

The reaction time varies as a function of the scale of the reaction and of the reaction temperature, but is generally between a few minutes and 48 hours.

The processes according to the invention are generally performed under standard pressure. However, it is also possible to work under elevated or reduced pressure.

For performance of the processes according to the invention, the starting materials required in each case are generally used in approximately equimolar amounts. However, it is also possible to use one of the components used in each case in a relatively large excess.

### Process A

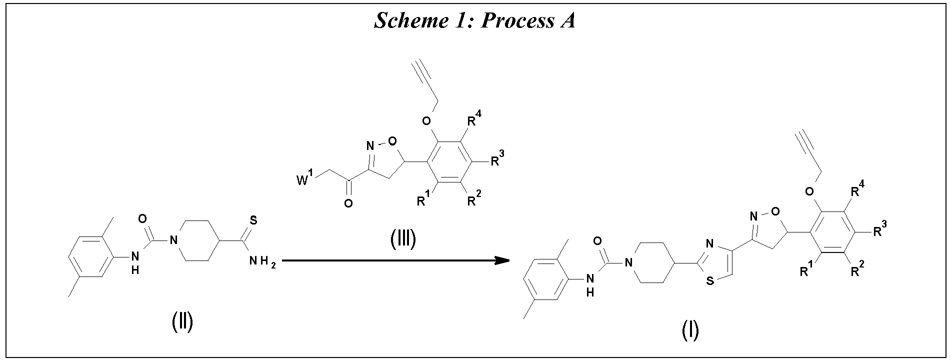

W¹ is a leaving group and the symbols R¹, R², R³, and R⁴ are each as defined in the description.

One means of preparing compounds of the formula **(I)** from corresponding compounds **(II)** by reaction with the compounds **(III)** is shown in Scheme 1 (*process A*).

The thiocarboxamide **(II)** is obtainable by methods known from the literature (see for example, WO 09/094407, WO 09/055514, WO 11/072207).
□-Halo ketones or corresponding ketones having a leaving group **(III)** (e.g. toluenesulphonyloxy ketones) are also obtainable by methods known from the literature (for examples see WO 08/013925, WO 13/098229, WO 12/055837).

The thiazoles **(I)** are obtained by a Hantzsch thiazole synthesis from the thiocarboxamides **(II)** and □-halo ketones or corresponding ketones having a leaving group **(III)** (see, for example, "Comprehensive Heterocyclic Chemistry", Pergamon Press, 1984; vol. 6, pages 235-363, "Comprehensive Heterocyclic Chemistry II", Pergamon Press, 1996; vol. 3, pages 373-474 and references cited therein, and WO 07/014290).

*Process A* is preferably performed using one or more diluents. In the performance of *process A,* inert organic solvents are a preferred option (for example N,N-dimethylformamide and ethanol).

If appropriate, an auxiliary base is used, for example triethylamine.

After the reaction has ended, the compounds (**I**) are separated from the reaction mixture by one of the customary separation techniques. If necessary, the compounds are purified by recrystallization or chromatography.

### Process B

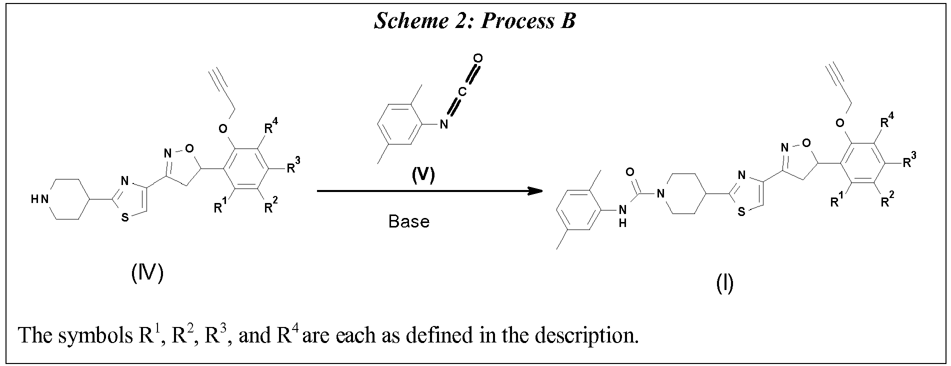

One means of preparing compounds of the formula **(I)** from corresponding compounds **(IV)** with the compounds **(V)** is shown in Scheme 2 (*process B*).

A compound with the general formula **(I)** can be synthesized analogously to methods described in the literature (see, for example WO 2012/055837), by a coupling reaction of a compound with the corresponding general formula **(IV)** with the isocyanate **(V),** optionally in the presence of an acid scavenger/base, for example triethylamine, 1,8-diazabicyclo[5.4.0]undec-7-ene or Hünig's base.

Isocyanate **(V)** is commercially available.

Compounds of formula **(IV)** are obtainable by methods known from the literature (see, for example WO 13/098229).

After the reaction has ended, the compounds (**I**) are separated from the reaction mixture by one of the customary separation techniques. If necessary, the compounds are purified by recrystallization or chromatography.

### Process C

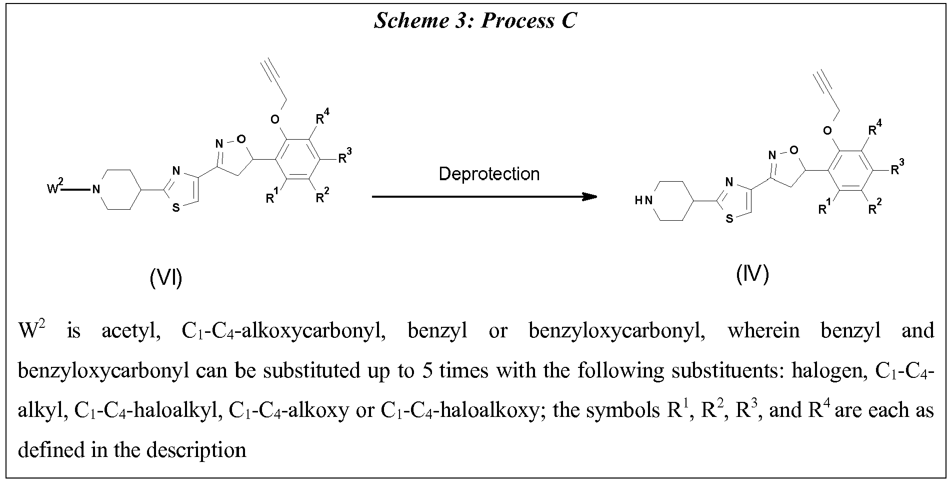

One means of preparing compounds of the formula **(IV)** from corresponding compounds **(VI)** is shown in Scheme 3 (*process C*).

A compound of the formula **(VI)** is converted to a compound of the formula **(IV)** by suitable methods for removing protecting groups described in the literature ("Protective Groups in Organic Synthesis"; Theodora W. Greene, Peter G. M. Wuts; Wiley-Interscience; Third Edition; 1999 494-653).

For example, *tert*-Butoxycarbonyl and benzyloxycarbonyl protecting groups can be removed in an acidic medium (for example with hydrochloric acid or trifluoroacetic acid, as for example described in WO 08/013925, WO 13/037768 and WO 13/098229). Acetyl protecting groups can be removed under basic conditions (for example with potassium carbonate or caesium carbonate). Benzylic protecting groups can be removed hydrogenolytically with hydrogen in the presence of a catalyst (for example palladium on activated carbon).

After the reaction has ended, the compounds **(IV)** are separated from the reaction mixture by one of the customary separation techniques. If necessary, the compounds are purified by recrystallization or chromatography, or can, if desired, also be used in the next step without prior purification. It is also possible to isolate the compound of the general formula **(IV)** as a salt, for example as a salt of hydrochloric acid or of trifluoroacetic acid.

### Process D

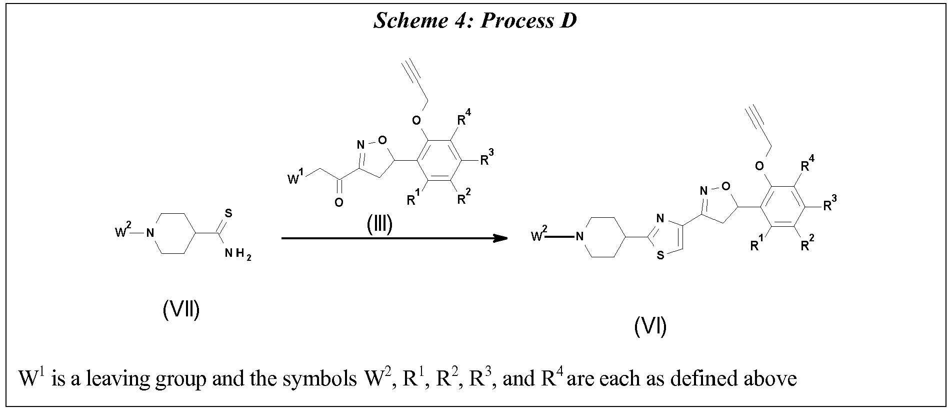

Another means of preparing the intermediate of the formula **(VI)** from corresponding compounds **(VII)** is shown in Scheme 4 (*process D*). Compounds **(VII)** are either commercially available or can be prepared by processes described in the literature (see, for example, WO 08/013622 and WO 07/014290). *Process D* is performed analogously to *process A* (Scheme 1).

### Process E

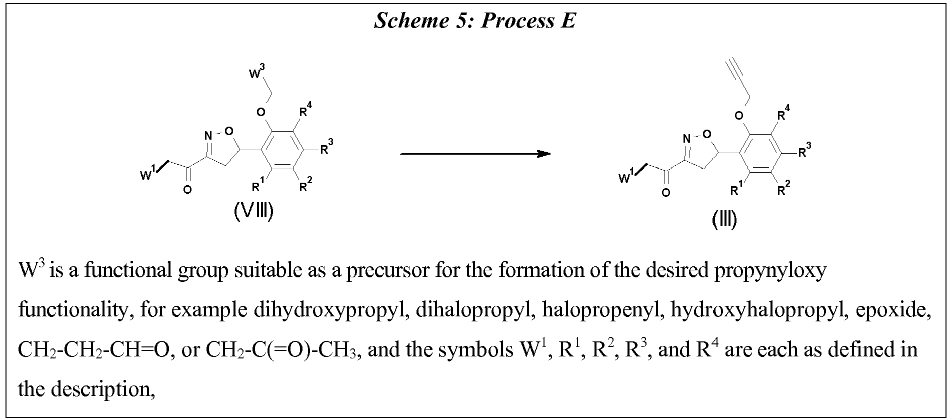

One means of preparing compounds of the formula (**III**) from corresponding compounds (**VIII**) by elimination reaction is shown in Scheme 5 (*process E*).

The alkynyloxy compounds (**III**) are obtained by reaction from precursors (**VIII**). There are several functional groups suitable for the formation of the desired alkyne functionality known in the literature, such as ketones (see, for example J. Heterocyclic Chem. 1982, 19, 1305-1308, Chem. Lett., 1998, 9, 863-864), dihalogenalkyls (see, for example Synlett 2010, 18, 2717-2720; Synthesis 2011, 15, 2377-2382), dihydroxyalkyls (e.g. via dihalogenalkyls, see, for example Tetrahedron Letters 2013, 54, 6420-6422), or haloalkenyls (see, for example J. Org. Chem. 1982, 47, 2484-7).

*Process E* is preferably performed using one or more diluents. In the performance of *process E,* inert organic solvents are a preferred option.

If necessary, *Process E* is performed using a base, for example triethylamine, 1,8-diazabicyclo[5.4.0]undec-7-ene (DBU) or tetra-N-butylammonium fluoride.

After the reaction has ended, the compounds (**III**) are separated from the reaction mixture by one of the customary separation techniques. If necessary, the compounds are purified by recrystallization or chromatography.

### Process F

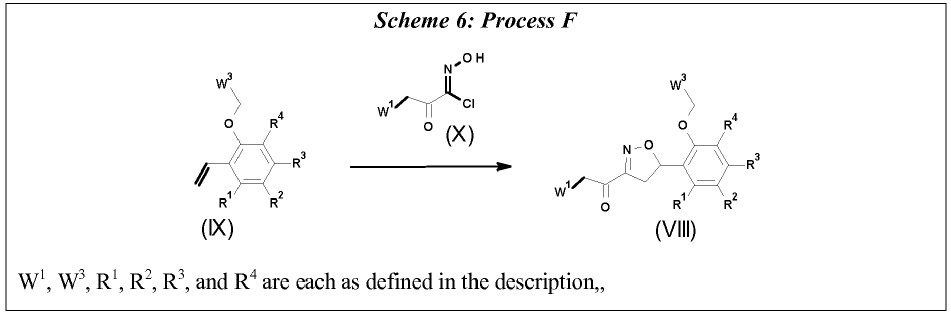

One means of preparing compounds of the formula (**VIII**) from corresponding compounds (**IX**) by reaction with the compounds (**X**) is shown in Scheme 6 (*process F*).

A compound of the general formula (**VIII**) is obtained from an alkene of the general formula (**IX**) and compound (**X**) by a cycloaddition reaction (see, for example, WO 08/013622 and Synthesis, 1987, 11, 998-1001).

The styrenes (**IX**) are commercially available or can be prepared from commercially available precursors by methods described in the literature (for example from aldehydes by a Wittig or Horner-Wadsworth-Emmons olefination: Chem. Rev. 1989, 89, 863-927 and Julia olefination: Tetrahedron Lett., 1973, 14, 4833-4836; Peterson olefination: J. Org. Chem. 1968, 33, 780; with the Bestmann-Ohira reagent: Synthesis 2004, 1, 59-62 or from hydroxystyrenes: Organometallics 2011, 30/15, 4144-4158, US2589378, DE825088). The corresponding aldehydes can be prepared from commercially available precursors by methods described in the literature (for example from the corresponding phenols: WO 13/163241; Synlett, 2006, 20, 3399-3402).

The chloroxims (**X**) can be prepared from commercially available precursors by methods described in the literature (for example from dihaloacetone by a nitrosation: Bull. Acad. Sci. USSR, Div. Chem. Sci. (Engl. Transl.) 1991, 40, 2.2, 438-441; WO 08/013925).

*Process F* is performed in the presence of a suitable base. Preferred bases are tertiary amines (e.g. triethylamine), and alkali metal or alkaline earth metal carbonates (for example potassium or sodium carbonate), hydrogencarbonates and phosphates.

*Process F* is preferably performed using one or more diluents. In the performance of *process F,* inert organic solvents are a preferred option (for example toluene and hexane). Water is likewise a possible solvent. Alternatively, *process F* can be performed in an excess of the alkene (**IX**).

The workup is efected by customary methods. If necessary, the compounds are purified by recrystallization or chromatography.

### Process G

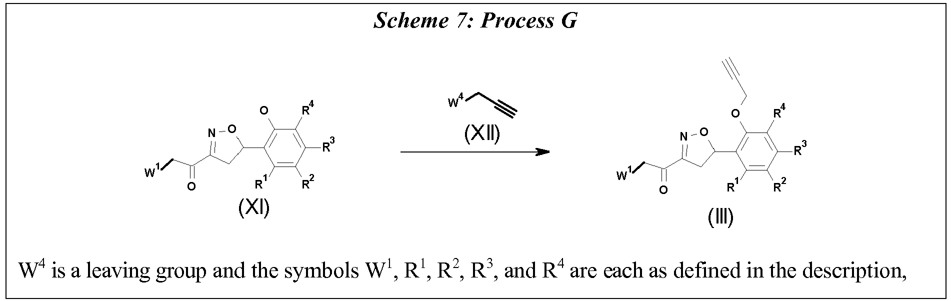

One means of preparing compounds of the formula (**III**) from corresponding compounds (**XI**) by reaction with the compounds (**XII**) is shown in Scheme 7 (*process G*).

The alkynyloxy compounds (**III**) are obtained from a phenole of the general formula (**XI**) and compound (**XII**) by a nucleophilic substitution reaction (see, for example, WO 11/076699).

*Process G* is preferably performed using one or more diluents. In the performance of *process G*, inert organic solvents are a preferred option.

The hydroxyphenylisoxazolines (**XI**) can be prepared from commercially available precursors by methods described in the literature (for example WO 08/013925, WO 09/094407).

If necessary, *Process G* is performed in the presence of a suitable base. Preferred bases are tertiary amines (e.g. triethylamine), and alkali metal or alkaline earth metal carbonates (for example potassium or sodium carbonate), hydrogencarbonates and phosphates.

After the reaction has ended, the compounds (**III**) are separated from the reaction mixture by one of the customary separation techniques. If necessary, the compounds are purified by recrystallization or chromatography.

### Process H

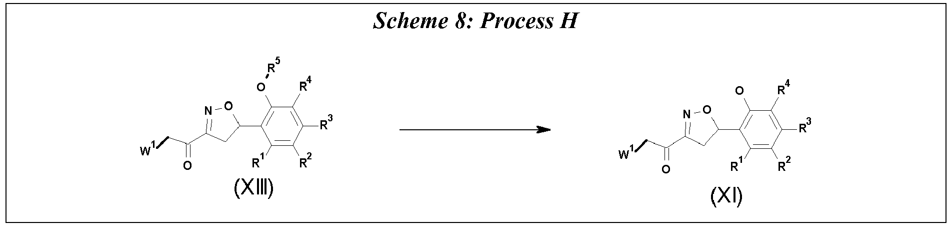

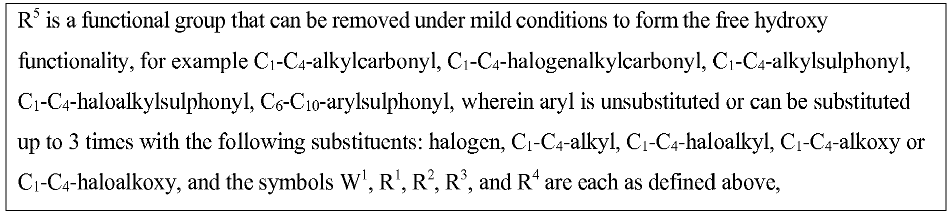

One means of preparing compounds of the formula (**XI**) from corresponding compounds (**XIII**) by cleavage reaction is shown in Scheme 8 (*process H*).

Phenols of formula (X**I**) are obtained from a compound of the general formula (**XI**) and compound (**XII**) by a hydrolysis reaction (see, for example, Synthesis, 2005, 12, 1971-1976; Organic Lett., 2004, 6(9), 1513-1514).

The hydroxyphenylisoxazolines (**XI**) can be prepared from commercially available precursors by methods described in the literature (for example WO 08/013925, WO 09/094407).

*Process H* is preferably performed using one or more diluents. In the performance of *process H*, inert organic solvents (e.g. ethers like THF) are a preferred option.

*Process H* is performed in the presence of a suitable base. Preferred bases are salts of bulky amines (e.g. lithium diisopropylamide), and alkali metal or alkaline earth metal hydroxides (for example potassium or sodium hydroxide).

After the reaction has ended, the compounds (**III**) are separated from the reaction mixture by one of the customary separation techniques. If necessary, the compounds are purified by recrystallization or chromatography.

### Process I

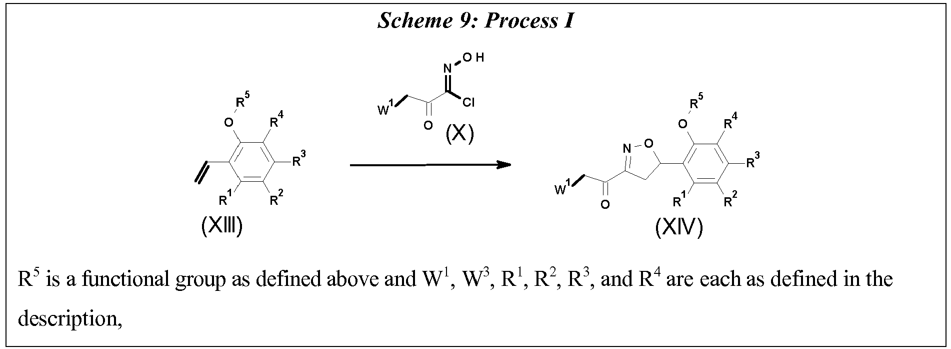

One means of preparing compounds of the formula (**XIV**) from corresponding compounds (**XIII**) by reaction with the compounds (**X**) is shown in Scheme 9 (*process I*).

A compound of the general formula (**XIV**) is obtained from a styrene of the general formula (**XIII**) and compound (**X**) by a cycloaddition reaction (see, for example, WO 08/013622 and Synthesis, 1987, 11, 998-1001).

The styrenes (**XIII**) are commercially available or can be prepared from commercially available precursors by methods described in the literature (for example from aldehydes by a Wittig or Horner-Wadsworth-Emmons olefination: Chem. Rev. 1989, 89, 863-927 and Julia olefination: Tetrahedron Lett., 1973, 14, 4833-4836; Peterson olefination: J. Org. Chem. 1968, 33, 780; with the Bestmann-Ohira reagent: Synthesis 2004, 1, 59-62 or from hydroxystyrenes: Organometallics 2011, 30/15, 4144-4158, US2589378, DE825088). The corresponding aldehydes can be prepared from commercially available precursors by methods described in the literature (for example from the corresponding phenols: WO 13/163241; Synlett, 2006, 20, 3399-3402).

The chloroxims (**X**) can be prepared from commercially available precursors by methods described in the literature (for example from dihaloacetone by a nitrosation: Bull. Acad. Sci. USSR, Div. Chem. Sci. (Engl. Transl.) 1991, 40, 2.2, 438-441; WO 08/013925).

*Process F* is performed in the presence of a suitable base. Preferred bases are tertiary amines (e.g. triethylamine), and alkali metal or alkaline earth metal carbonates (for example potassium or sodium carbonate), hydrogencarbonates and phosphates.

*Process F* is preferably performed using one or more diluents. In the performance of *process F,* inert organic solvents are a preferred option (for example toluene and hexane). Water is likewise a possible solvent. Alternatively, *process F* can be performed in an excess of the styrenes (**XIII**).

The workup is efected by customary methods. If necessary, the compounds are purified by recrystallization or chromatography.

Furthermore, it is also recognized that some reagents and reaction conditions described above for preparation of compounds of the formula **(I)** may not be compatible with particular functionalities present in the intermediate compounds. In these cases, the introduction of protection/deprotection sequences or of mutual conversions of functional groups into the synthesis helps to obtain the desired products. The use and selection of the protecting groups is obvious to the person skilled in the art of chemical synthesis (see, for example, "Protective Groups in Organic Synthesis"; Third Edition; 494-653, and literature cited therein). The person skilled in the art will recognize that, in some cases, after the introduction of a given reagent as shown in an individual scheme, it may be necessary to perform additional routine synthesis steps not described individually in order to complete the synthesis of compounds of the formula **(I).** The person skilled in the art will likewise recognize that it may be necessary to perform a combination of the steps illustrated in the above schemes in a sequence other than the implied sequence shown specifically, in order to prepare the compounds of the formula **(I).**

The workup is carried out by customary methods. If necessary, the compounds are purified by recrystallization or chromatography.

Compounds of the formula (**VI**) are new, 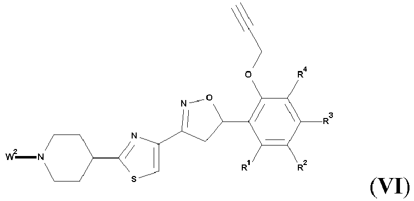 in which the radicals R¹, R², R³, and R⁴ are each as defined in the description above and in which W² is defined as follows:
W² is acetyl, C₁-C₄-alkoxycarbonyl, benzyl or benzyloxycarbonyl, wherein benzyl and benzyloxycarbonyl can be substituted up to 5 times with the following substituents: halogen, C₁-C₄-alkyl, C₁-C₄-haloalkyl, C₁-C₄-alkoxy or C₁-C₄-haloalkoxy.

W² is preferably acetyl, methoxycarbonyl, ethoxycarbonyl, benzyl or benzyloxycarbonyl, wherein benzyl and benzyloxycarbonyl can be substituted 1, 2, or 3 times with the following substituents: fluoro, chloro, methyl, ethyl, n-propyl, difluoromethyl, trifluoromethyl, trichloromethyl, CH₂CF₃, methoxy, ethoxy, n-propoxy, t-butyloxy, difluoromethoxy, trifluoromethoxy, OCH₂CHF₂, OCF₂CF₃, or OCH₂CF₃.

W² is more preferably acetyl, methoxycarbonyl, ethoxycarbonyl, benzyl or benzyloxycarbonyl, wherein benzyl and benzyloxycarbonyl can be substituted 1 or 2 times with the following substituents: fluoro, chloro, methyl, ethyl, difluoromethyl, trifluoromethyl, methoxy, ethoxy, difluoromethoxy, or trifluoromethoxy.

Compounds of the formula (**VIII**) are new, 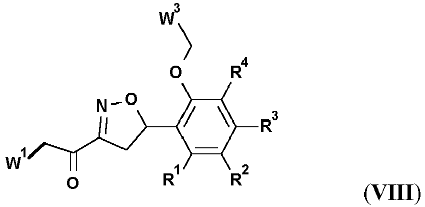 in which the radical W³ is dihydroxypropyl, dihalopropyl, halopropenyl, hydroxyhalopropyl, epoxide, CH₂-CH₂-CH=O, or CH₂-C(=O)-CH₃, and the symbols W¹, R¹, R², R³, and R⁴ are each as defined in the description above.

Compounds of the formula (**XIII**) are new, 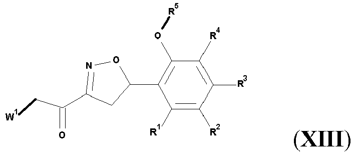 in which the radicals W¹, R¹, R², R³, and R⁴ are each as defined in the description above and in which R⁵ is defined as follows:
R⁵ is C₁-C₄-alkylcarbonyl, C₁-C₄-halogenalkylcarbonyl, C₂-C₄-alkylsulphonyl, C₁-C₄-haloalkylsulphonyl, C₆-C₁₀-arylsulphonyl, wherein aryl is unsubstituted or can be substituted up to 3 times with the following substituents: halogen, C₁-C₄-alkyl, C₁-C₄-haloalkyl, C₁-C₄-alkoxy or C₁-C₄-haloalkoxy.

R⁵ is preferably acetyl, ethylcarbonyl, C₁-C₂-halogenalkylcarbonyl, ethylsulphonyl, C₁-C₂-haloalkylsulphonyl, phenylsulphonyl, wherein phenyl is unsubstituted or can be substituted once or twice with the following substituents: fluoro, chloro, methyl, ethyl, trifluoromethyl, difluoromethyl, methoxy, ethoxy or trifluoromethoxy.

R⁵ is more preferably acetyl, ethylcarbonyl, trifluoroacetyl, ethylsulphonyl, trifluoromethylsulphonyl, phenylsulphonyl or (4-methylphenyl)sulfonyl.

The invention also relates to a method for controlling unwanted microorganisms, characterized in that the inventive Phenylpiperidinecarboxamide derivatives are applied to the microorganisms and/or in their habitat.

The invention further relates to seed which has been treated with at least one inventive phenyl ureaderivative.

The invention finally provides a method for protecting seed against unwanted microorganisms by using seed treated with at least one phenyl ureaderivative according to the present invention.

The inventive substances have potent microbicidal activity and can be used for control of unwanted microorganisms, such as fungi and bacteria, in crop protection and in the protection of materials.

The inventive Phenylpiperidinecarboxamide derivatives of the formula **(I)** have very good fungicidal properties and can be used in crop protection, for example for control of Plasmodiophoromycetes, Oomycetes, Chytridiomycetes, Zygomycetes, Ascomycetes, Basidiomycetes and Deuteromycetes.

Bactericides can be used in crop protection, for example, for control of Pseudomonadaceae, Rhizobiaceae, Enterobacteriaceae, Corynebacteriaceae and Streptomycetaceae.

The inventive fungicidal compositions can be used for curative or protective control of phytopathogenic fungi. The invention therefore also relates to curative and protective methods for controlling phytopathogenic fungi by the use of the inventive active ingredients or compositions, which are applied to the seed, the plant or plant parts, the fruit or the soil in which the plants grow.

The inventive compositions for controlling phytopathogenic fungi in crop protection comprise an effective but non-phytotoxic amount of the inventive active ingredients. An "effective but non-phytotoxic amount" means an amount of the inventive composition which is sufficient to control the fungal disease of the plant in a satisfactory manner or to eradicate the fungal disease completely, and which, at the same time, does not cause any significant symptoms of phytotoxicity. In general, this application rate may vary within a relatively wide range. It depends on several factors, for example on the fungus to be controlled, the plant, the climatic conditions and the ingredients of the inventive compositions.

All plants and plant parts can be treated in accordance with the invention. Plants are understood here to mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the transgenic plants and including the plant cultivars which are protectable and non-protectable by plant breeders' rights. Plant parts are understood to mean all parts and organs of plants above and below the ground, such as shoot, leaf, flower and root, examples of which include leaves, needles, stalks, stems, flowers, fruit bodies, fruits and seeds, and also roots, tubers and rhizomes. The plant parts also include harvested material and vegetative and generative propagation material, for example cuttings, tubers, rhizomes, slips and seeds.

### Composition / Formulation

The present invention further relates to a crop protection composition for controlling harmful microorganisms, especially unwanted fungi and bacteria, comprising an effective and non-phytotoxic amount of the inventive active ingredients. These are preferably fungicidal compositions which comprise agriculturally suitable auxiliaries, solvents, carriers, surfactants or extenders.

In the context of the present invention, "control of harmful microorganisms" means a reduction in infestation by harmful microorganisms, compared with the untreated plant measured as fungicidal efficacy, preferably a reduction by 25-50 %, compared with the untreated plant (100%), more preferably a reduction by 40-79 %, compared with the untreated plant (100 %); even more preferably, the infection by harmful microorganisms is entirely suppressed (by 70-100 %). The control may be curative, i.e. for treatment of already infected plants, or protective, for protection of plants which have not yet been infected.

An "effective but non-phytotoxic amount" means an amount of the inventive composition which is sufficient to control the fungal disease of the plant in a satisfactory manner or to eradicate the fungal disease completely, and which, at the same time, does not cause any significant symptoms of phytotoxicity. In general, this application rate may vary within a relatively wide range. It depends on several factors, for example on the fungus to be controlled, the plant, the climatic conditions and the ingredients of the inventive compositions.

Suitable organic solvents include all polar and non-polar organic solvents usually employed for formulation purposes. Preferable the solvents are selected from ketones, e.g. methyl-isobutyl-ketone and cyclohexanone, amides, e.g. dimethyl formamide and alkanecarboxylic acid amides, e.g. N,N-dimethyl decaneamide and N,N-dimethyl octanamide, furthermore cyclic solvents, e.g. N-methylpyrrolidone, N-octyl-pyrrolidone, N-dodecyl-pyrrolidone, N-octyl-caprolactame, N-dodecyl-caprolactame and butyrolactone, furthermore strong polar solvents, e.g. dimethylsulfoxide, and aromatic hydrocarbons, e.g. xylol, Solvesso™, mineral oils, e.g. white spirit, petroleum, alkyl benzenes and spindle oil, also esters, e.g. propyleneglycol-monomethylether acetate, adipic acid dibutylester, acetic acid hexylester, acetic acid heptylester, citric acid tri-n-butylester and phthalic acid di-n-butylester, and also alkohols, e.g. benzyl alcohol and 1 -methoxy-2-propanol.

According to the invention, a carrier is a natural or synthetic, organic or inorganic substance with which the active ingredients are mixed or combined for better applicability, in particular for application to plants or plant parts or seed. The carrier which may be solid or liquid, is generally inert and should be suitable for use in agriculture.

Useful solid or liquid carriers include: for example ammonium salts and natural rock dusts, such as kaolins, clays, talc, chalk, quartz, attapulgite, montmorillonite or diatomaceous earth, and synthetic rock dusts, such as finely divided silica, alumina and natural or synthetic silicates, resins, waxes, solid fertilizers, water, alcohols, especially butanol, organic solvents, mineral and vegetable oils, and derivatives thereof. Mixtures of such carriers can likewise be used.

Suitable solid filler and carrier include inorganic particles, e.g. carbonates, silikates, sulphates and oxides with an average particle size of between 0.005 and 20 µm, preferably of between 0.02 to 10 µm, for example ammonium sulphate, ammonium phosphate, urea, calcium carbonate, calcium sulphate, magnesium sulphate, magnesium oxide, aluminium oxide, silicium dioxide, so-called fine-particle silica, silica gels, natural or synthetic silicates, and alumosilicates and plant products like cereal flour, wood powder/sawdust and cellulose powder.

Useful solid carriers for granules include: for example crushed and fractionated natural rocks such as calcite, marble, pumice, sepiolite, dolomite, and synthetic granules of inorganic and organic meals, and also granules of organic material such as sawdust, coconut shells, maize cobs and tobacco stalks.

Useful liquefied gaseous extenders or carriers are those liquids which are gaseous at standard temperature and under standard pressure, for example aerosol propellants such as halohydrocarbons, and also butane, propane, nitrogen and carbon dioxide.

In the formulations, it is possible to use tackifiers such as carboxymethylcellulose, and natural and synthetic polymers in the form of powders, granules or latices, such as gum arabic, polyvinyl alcohol and polyvinyl acetate, or else natural phospholipids, such as cephalins and lecithins, and synthetic phospholipids. Further additives may be mineral and vegetable oils.

If the extender used is water, it is also possible to employ, for example, organic solvents as auxiliary solvents. Useful liquid solvents are essentially: aromatics such as xylene, toluene or alkylnaphthalenes, chlorinated aromatics and chlorinated aliphatic hydrocarbons such as chlorobenzenes, chloroethylenes or dichloromethane, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, and also water.

Suitable surfactants (adjuvants, emulsifiers, dispersants, protective colloids, wetting agent and adhesive) include all common ionic and non-ionic substances, for example ethoxylated nonylphenols, polyalkylene glycolether of linear or branched alcohols, reaction products of alkyl phenols with ethylene oxide and/or propylene oxide, reaction products of fatty acid amines with ethylene oxide and/or propylene oxide, furthermore fattic acid esters, alkyl sulfonates, alkyl sulphates, alkyl ethersulphates, alkyl etherphosphates, arylsulphate, ethoxylated arylalkylphenols, e.g. tristyryl-phenol-ethoxylates, furthermore ethoxylated and propoxylated arylalkylphenols like sulphated or phosphated arylalkylphenol-ethoxylates and -ethoxy- and -propoxylates. Further examples are natural and synthetic, water soluble polymers, e.g. lignosulphonates, gelatine, gum arabic, phospholipides, starch, hydrophobic modified starch and cellulose derivatives, in particular cellulose ester and cellulose ether, further polyvinyl alcohol, polyvinyl acetate, polyvinyl pyrrolidone, polyacrylic acid, polymethacrylic acid and co-polymerisates of (meth)acrylic acid and (meth)acrylic acid esters, and further co-polymerisates of methacrylic acid and methacrylic acid esters which are neutralized with alkalimetal hydroxide and also condensation products of optionally substituted naphthalene sulfonic acid salts with formaldehyde. The presence of a surfactant is necessary if one of the active ingredients and/or one of the inert carriers is insoluble in water and when application is effected in water. The proportion of surfactants is between 5 and 40 per cent by weight of the inventive composition.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

Antifoams which may be present in the formulations include e.g. silicone emulsions, longchain alcohols, fattiy acids and their salts as well as fluoroorganic substances and mixtures therof.

Examples of thickeners are polysaccharides, e.g. xanthan gum or veegum, silicates, e.g. attapulgite, bentonite as well as fine-particle silica.

If appropriate, it is also possible for other additional components to be present, for example protective colloids, binders, adhesives, thickeners, thixotropic substances, penetrants, stabilizers, sequestrants, complexing agents. In general, the active ingredients can be combined with any solid or liquid additive commonly used for formulation purposes.

The inventive active ingredients or compositions can be used as such or, depending on their particular physical and/or chemical properties, in the form of their formulations or the use forms prepared therefrom, such as aerosols, capsule suspensions, cold-fogging concentrates, warm-fogging concentrates, encapsulated granules, fine granules, flowable concentrates for the treatment of seed, ready-to-use solutions, dustable powders, emulsifiable concentrates, oil-in-water emulsions, water-in-oil emulsions, macrogranules, microgranules, oil-dispersible powders, oil-miscible flowable concentrates, oil-miscible liquids, gas (under pressure), gas generating product, foams, pastes, pesticide coated seed, suspension concentrates, suspoemulsion concentrates, soluble concentrates, suspensions, wettable powders, soluble powders, dusts and granules, water-soluble and water-dispersible granules or tablets, water-soluble and water-dispersible powders for the treatment of seed, wettable powders, natural products and synthetic substances impregnated with active ingredient, and also microencapsulations in polymeric substances and in coating materials for seed, and also ULV cold-fogging and warm-fogging formulations.

The inventive compositions include not only formulations which are already ready for use and can be applied with a suitable apparatus to the plant or the seed, but also commercial concentrates which have to be diluted with water prior to use. Customary applications are for example dilution in water and subsequent spraying of the resulting spray liquor, application after dilution in oil, direct application without dilution, seed treatment or soil application of granules.

The inventive compositions and formulations generally contain between 0.05 and 99 % by weight, 0.01 and 98 % by weight, preferably between 0.1 and 95 % by weight, more preferably between 0.5 and 90 % of active ingredient, most preferably between 10 and 70 % by weight. For special applications, e.g. for protection of wood and derived timber products the inventive compositions and formulations generally contain between 0.0001 and 95 % by weight, preferably 0.001 to 60 % by weight of active ingredient.

The contents of active ingredient in the application forms prepared from the commercial formulations may vary in a broad range. The concentration of the active ingredients in the application forms is generally between 0.000001 to 95 % by weight, preferably between 0.0001 and 2 % by weight.

The formulations mentioned can be prepared in a manner known per se, for example by mixing the active ingredients with at least one customary extender, solvent or diluent, adjuvant, emulsifier, dispersant, and/or binder or fixative, wetting agent, water repellent, if appropriate desiccants and UV stabilizers and, if appropriate, dyes and pigments, antifoams, preservatives, inorganic and organic thickeners, adhesives, gibberellins and also further processing auxiliaries and also water. Depending on the formulation type to be prepared further processing steps are necessary, e.g. wet grinding, dry grinding and granulation.

The inventive active ingredients may be present as such or in their (commercial) formulations and in the use forms prepared from these formulations as a mixture with other (known) active ingredients, such as insecticides, attractants, sterilants, bactericides, acaricides, nematicides, fungicides, growth regulators, herbicides, fertilizers, safeners and/or semiochemicals.

The inventive treatment of the plants and plant parts with the active ingredients or compositions is effected directly or by action on their surroundings, habitat or storage space by the customary treatment methods, for example by dipping, spraying, atomizing, irrigating, evaporating, dusting, fogging, broadcasting, foaming, painting, spreading-on, watering (drenching), drip irrigating and, in the case of propagation material, especially in the case of seeds, also by dry seed treatment, wet seed treatment, slurry treatment, incrustation, coating with one or more coats, etc. It is also possible to deploy the active ingredients by the ultra-low volume method or to inject the active ingredient preparation or the active ingredient itself into the soil.

### Plant/Crop Protection

The inventive active ingredients or compositions have potent microbicidal activity and can be used for control of unwanted microorganisms, such as fungi and bacteria, in crop protection and in the protection of materials.

The invention also relates to a method for controlling unwanted microorganisms, characterized in that the inventive active ingredients are applied to the phytopathogenic fungi, phytopathogenic bacteria and/or their habitat.

Fungicides can be used in crop protection for control of phytopathogenic fungi. They are characterized by an outstanding efficacy against a broad spectrum of phytopathogenic fungi, including soilborne pathogens, which are in particular members of the classes *Plasmodiophoromycetes, Peronosporomycetes* (Syn. *Oomycetes*), *Chytridiomycetes*, *Zygomycetes*, *Ascomycetes, Basidiomycetes* and *Deuteromycetes* (Syn. *Fungi imperfecti*). Some fungicides are systemically active and ca be used in plant protection as foliar, seed dressing or soil fungicide. Furthermore, they are suitable for combating fungi, which inter alia infest wood or roots of plant.

Bactericides can be used in crop protection for control of *Pseudomonadaceae*, *Rhizobiaceae, Enterobacteriaceae, Corynebacteriaceae* and *Streptomycetaceae.*

Non-limiting examples of pathogens of fungal diseases which can be treated in accordance with the invention include:
diseases caused by powdery mildew pathogens, for example *Blumeria* species, for example *Blumeria* graminis; *Podosphaera* species, for example *Podosphaera leucotricha*; *Sphaerotheca* species, for example *Sphaerotheca fuliginea*; *Uncinula* species, for example *Uncinula necator*;
diseases caused by rust disease pathogens, for example *Gymnosporangium* species, for example *Gymnosporangium sabinae*; *Hemileia* species, for example *Hemileia vastatrix*; *Phakopsora* species, for example *Phakopsora pachyrhizi* and *Phakopsora meibomiae*; *Puccinia* species, for example *Puccinia recondite, P. triticina*, *P. graminis* or *P. striiformis*; *Uromyces* species, for example *Uromyces appendiculatus*;
diseases caused by pathogens from the group of the *Oomycetes*, for example *Albugo* species, for example *Algubo candida*; *Bremia* species, for example *Bremia lactucae*; *Peronospora* species, for example *Peronospora pisi* or *P. brassicae*; Phytophthora species, for example *Phytophthora infestans*; *Plasmopara* species, for example *Plasmopara viticola*; *Pseudoperonospora* species, for example *Pseudoperonospora humuli* or *Pseudoperonospora cubensis*; Pythium species, for example *Pythium ultimum*;
leaf blotch diseases and leaf wilt diseases caused, for example, by *Alternaria* species, for example *Alternaria solani*; *Cercospora* species, for example *Cercospora beticola*; *Cladiosporium* species, for example *Cladiosporium cucumerinum*; *Cochliobolus* species, for example *Cochliobolus sativus* (conidia form: Drechslera, Syn: Helminthosporium), *Cochliobolus miyabeanus*; *Colletotrichum* species, for example *Colletotrichum lindemuthanium*; *Cycloconium* species, for example *Cycloconium oleaginum*; *Diaporthe* species, for example *Diaporthe citri*; *Elsinoe* species, for example *Elsinoe fawcettii*; *Gloeosporium* species, for example *Gloeosporium laeticolor*; *Glomerella* species, for example *Glomerella cingulata*; *Guignardia* species, for example *Guignardia bidwelli*; *Leptosphaeria* species, for example *Leptosphaeria maculans*, *Leptosphaeria nodorum*; *Magnaporthe* species, for example *Magnaporthe grisea*; *Microdochium* species, for example *Microdochium nivale*; *Mycosphaerella* species, for example *Mycosphaerella graminicola, M. arachidicola* and *M. ftjiensis*; *Phaeosphaeria* species, for example *Phaeosphaeria nodorum*; *Pyrenophora* species, for example *Pyrenophora teres, Pyrenophora tritici repentis*; *Ramularia* species, for example *Ramularia collocygni, Ramularia areola*; *Rhynchosporium* species, for example *Rhynchosporium secalis*; *Septoria* species, for example *Septoria apii*, *Septoria lycopersii*; *Typhula* species, for example *Typhula incarnata*; *Venturia* species, for example *Venturia inaequalis*;
root and stem diseases caused, for example, by *Corticium* species, for example *Corticium graminearum*; *Fusarium* species, for example *Fusarium oxysporum*; *Gaeumannomyces* species, for example *Gaeumannomyces graminis*; *Rhizoctonia* species, such as, for example *Rhizoctonia solani*; *Sarocladium* diseases caused for example by *Sarocladium oryzae*; *Sclerotium* diseases caused for example by *Sclerotium oryzae*; *Tapesia* species, for example *Tapesia acuformis*; *Thielaviopsis* species, for example *Thielaviopsis basicola*;
ear and panicle diseases (including corn cobs) caused, for example, by *Alternaria* species, for example *Alternaria* spp.; *Aspergillus* species, for example *Aspergillus flavus*; *Cladosporium* species, for example *Cladosporium cladosporioides*; *Claviceps* species, for example *Claviceps purpurea*; *Fusarium* species, for example *Fusarium culmorum*; *Gibberella* species, for example *Gibberella zeae*; *Monographella* species, for example *Monographella nivalis*; *Septoria* species, for example *Septoria nodorum*;
diseases caused by smut fungi, for example *Sphacelotheca* species, for example *Sphacelotheca reiliana*; *Tilletia* species, for example *Tilletia caries, T. controversa*; *Urocystis* species, for example *Urocystis occulta*; *Ustilago* species, for example *Ustilago nuda, U. nuda tritici*;
fruit rot caused, for example, by *Aspergillus* species, for example *Aspergillus flavus*; *Botrytis* species, for example *Botrytis cinerea*; *Penicillium* species, for example *Penicillium expansum* and *P. purpurogenum*; *Sclerotinia* species, for example *Sclerotinia sclerotiorum*; *Verticilium* species, for example *Verticilium alboatrum*;
seed and soilborne decay, mould, wilt, rot and damping-off diseases caused, for example, by *Alternaria* species, caused for example by *Alternaria brassicicola*; *Aphanomyces* species, caused for example by *Aphanomyces euteiches*; *Ascochyta* species, caused for example by *Ascochyta lentis*; *Aspergillus* species, caused for example by *Aspergillus flavus*; *Cladosporium* species, caused for example by *Cladosporium herbarum*; *Cochliobolus* species, caused for example by *Cochliobolus sativus*; (Conidiaform: Drechslera, Bipolaris Syn: Helminthosporium); *Colletotrichum* species, caused for example by *Colletotrichum coccodes*; *Fusarium* species, caused for example by *Fusarium culmorum*; *Gibberella* species, caused for example by *Gibberella zeae*; *Macrophomina* species, caused for example by *Macrophomina phaseolina*; *Monographella* species, caused for example by *Monographella nivalis*; *Penicillium* species, caused for example by *Penicillium expansum*; *Phoma* species, caused for example by *Phoma lingam*; *Phomopsis* species, caused for example by *Phomopsis sojae*; *Phytophthora* species, caused for example by *Phytophthora cactorum*; *Pyrenophora* species, caused for example by *Pyrenophora graminea*; *Pyricularia* species, caused for example by *Pyricularia oryzae*; *Pythium* species, caused for example by *Pythium ultimum*; *Rhizoctonia* species, caused for example by *Rhizoctonia solani*; *Rhizopus* species, caused for example by *Rhizopus oryzae*; *Sclerotium* species, caused for example by *Sclerotium rolfsii*; *Septoria* species, caused for example by *Septoria nodorum*; *Typhula* species, caused for example by *Typhula incarnata*; *Verticillium* species, caused for example by *Verticillium dahliae*;
cancers, galls and witches' broom caused, for example, by *Nectria* species, for example *Nectria galligena*;
wilt diseases caused, for example, by *Monilinia* species, for example *Monilinia laxa*;
leaf blister or leaf curl diseases caused, for example, by *Exobasidium* species, for example *Exobasidium vexans;*
*Taphrina* species, for example *Taphrina deformans*;
decline diseases of wooden plants caused, for example, by Esca disease, caused for example by *Phaemoniella clamydospora, Phaeoacremonium aleophilum* and *Fomitiporia mediterranea*; Eutypa dyeback, caused for example by *Eutypa lata*; Ganoderma diseases caused for example by *Ganoderma boninense*; Rigidoporus diseases caused for example by *Rigidoporus lignosus*;
diseases of flowers and seeds caused, for example, by *Botrytis* species, for example *Botrytis cinerea*;
diseases of plant tubers caused, for example, by *Rhizoctonia* species, for example *Rhizoctonia solani*; *Helminthosporium* species, for example *Helminthosporium solani*;
Club root caused, for example, by *Plasmodiophora* species, for example *Plamodiophora brassicae*;
diseases caused by bacterial pathogens, for example *Xanthomonas* species, for example *Xanthomonas campestris pv. oryzae*; *Pseudomonas* species, for example *Pseudomonas syringae pv. lachrymans*; *Erwinia* species, for example *Erwinia amylovora.*

The following diseases of soya beans can be controlled with preference:
Fungal diseases on leaves, stems, pods and seeds caused, for example, by *Alternaria* leaf spot (*Alternaria spec. atrans tenuissima*), Anthracnose (*Colletotrichum gloeosporoides dematium var. truncatum*), brown spot (*Septoria glycines*), cercospora leaf spot and blight (*Cercospora kikuchii*), choanephora leaf blight (*Choanephora infundibulifera trispora* (Syn.)), dactuliophora leaf spot (*Dactuliophora glycines*), downy mildew (*Peronospora manshurica*), drechslera blight (*Drechslera glycini*), frogeye leaf spot (*Cercospora sojina*), leptosphaerulina leaf spot (*Leptosphaerulina trifolii*), phyllostica leaf spot (*Phyllosticta sojaecola*), pod and stem blight (*Phomopsis sojae*), powdery mildew (*Microsphaera diffusa*), pyrenochaeta leaf spot (*Pyrenochaeta glycines*), rhizoctonia aerial, foliage, and web blight (*Rhizoctonia solani*), rust (*Phakopsora pachyrhizi*, *Phakopsora meibomiae*), scab (*Sphaceloma glycines*), stemphylium leaf blight (*Stemphylium botryosum*), target spot (*Corynespora cassiicola*).

Fungal diseases on roots and the stem base caused, for example, by black root rot (*Calonectria crotalariae*), charcoal rot (*Macrophomina phaseolina*), fusarium blight or wilt, root rot, and pod and collar rot (*Fusarium oxysporum, Fusarium orthoceras*, *Fusarium semitectum*, *Fusarium equiseti*), mycoleptodiscus root rot (*Mycoleptodiscus terrestris*), neocosmospora (*Neocosmospora vasinfecta*), pod and stem blight (*Diaporthe phaseolorum*), stem canker (*Diaporthe phaseolorum var. caulivora*), phytophthora rot (*Phytophthora megasperma*), brown stem rot (*Phialophora gregata*), pythium rot (*Pythium aphanidermatum*, *Pythium irregulare*, *Pythium debaryanum*, *Pythium myriotylum*, *Pythium ultimum*), rhizoctonia root rot, stem decay, and damping-off (*Rhizoctonia solani*), sclerotinia stem decay (*Sclerotinia sclerotiorum*), sclerotinia southern blight (*Sclerotinia rolfsii*), thielaviopsis root rot (*Thielaviopsis basicola*).

The inventive fungicidal compositions can be used for curative or protective/preventive control of phytopathogenic fungi. The invention therefore also relates to curative and protective methods for controlling phytopathogenic fungi by the use of the inventive active ingredients or compositions, which are applied to the seed, the plant or plant parts, the fruit or the soil in which the plants grow.

The fact that the active ingredients are well tolerated by plants at the concentrations required for controlling plant diseases allows the treatment of above-ground parts of plants, of propagation stock and seeds, and of the soil.

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners and seeds also belong to plant parts.

The inventive active ingredients, when they are well tolerated by plants, have favourable homeotherm toxicity and are well tolerated by the environment, are suitable for protecting plants and plant organs, for enhancing harvest yields, for improving the quality of the harvested material. They can preferably be used as crop protection compositions. They are active against normally sensitive and resistant species and against all or some stages of development.

Plants which can be treated in accordance with the invention include the following main crop plants: maize, soya bean, alfalfa, cotton, sunflower, *Brassica* oil seeds such as *Brassica napus* (e.g. canola, rapeseed), *Brassica rapa, B. juncea* (e.g. (field) mustard) and *Brassica carinata*, *Arecaceae sp.* (e.g. oilpalm, coconut), rice, wheat, sugar beet, sugar cane, oats, rye, barley, millet and sorghum, triticale, flax, nuts, grapes and vine and various fruit and vegetables from various botanic taxa, e.g. *Rosaceae sp.* (e.g. pome fruits such as apples and pears, but also stone fruits such as apricots, cherries, almonds, plums and peaches, and berry fruits such as strawberries, raspberries, red and black currant and gooseberry), *Ribesioidae sp., Juglandaceae sp., Betulaceae sp., Anacardiaceae sp., Fagaceae sp., Moraceae sp., Oleaceae sp.* (e.g. olive tree), *Actinidaceae sp., Lauraceae sp.* (e.g. avocado, cinnamon, camphor), *Musaceae sp.* (e.g. banana trees and plantations), *Rubiaceae sp.* (e.g. coffee), *Theaceae sp.* (e.g. tea), *Sterculiceae sp., Rutaceae sp.* (e.g. lemons, oranges, mandarins and grapefruit); *Solanaceae sp.* (e.g. tomatoes, potatoes, peppers, capsicum, aubergines, tobacco), *Liliaceae sp., Compositae sp.* (e.g. lettuce, artichokes and chicory - including root chicory, endive or common chicory), *Umbelliferae sp.* (e.g. carrots, parsley, celery and celeriac), *Cucurbitaceae sp.* (e.g. cucumbers - including gherkins, pumpkins, watermelons, calabashes and melons), *Alliaceae sp.* (e.g. leeks and onions), *Cruciferae sp.* (e.g. white cabbage, red cabbage, broccoli, cauliflower, Brussels sprouts, pak choi, kohlrabi, radishes, horseradish, cress and chinese cabbage), *Leguminosae sp.* (e.g. peanuts, peas, lentils and beans - e.g. common beans and broad beans), *Chenopodiaceae sp.* (e.g. Swiss chard, fodder beet, spinach, beetroot), *Linaceae sp.* (e.g. hemp), *Cannabeacea sp.* (e.g. cannabis), *Malvaceae sp.* (e.g. okra, cocoa), *Papaveraceae* (e.g. poppy), *Asparagaceae* (e.g. asparagus); useful plants and ornamental plants in the garden and woods including turf, lawn, grass and *Stevia rebaudiana*; and in each case genetically modified types of these plants.

### Seed Treatment

The invention further comprises a method for treating seed.

The invention further relates to seed which has been treated by one of the methods described in the previous paragraph. The inventive seeds are employed in methods for the protection of seed from harmful microorganisms. In these methods, seed treated with at least one inventive active ingredient is used.

The inventive active ingredients or compositions are also suitable for treating seed. A large part of the damage to crop plants caused by harmful organisms is triggered by the infection of the seed during storage or after sowing, and also during and after germination of the plant. This phase is particularly critical since the roots and shoots of the growing plant are particularly sensitive, and even minor damage may result in the death of the plant. There is therefore a great interest in protecting the seed and the germinating plant by using appropriate compositions.

The control of phytopathogenic fungi by treating the seed of plants has been known for a long time and is the subject of constant improvements. However, the treatment of seed entails a series of problems which cannot always be solved in a satisfactory manner. For instance, it is desirable to develop methods for protecting the seed and the germinating plant, which dispense with, or at least significantly reduce, the additional deployment of crop protection compositions after planting or after emergence of the plants. It is also desirable to optimize the amount of the active ingredient used so as to provide the best possible protection for the seed and the germinating plant from attack by phytopathogenic fungi, but without damaging the plant itself by the active ingredient employed. In particular, methods for the treatment of seed should also take account of the intrinsic fungicidal properties of transgenic plants in order to achieve optimal protection of the seed and the germinating plant with a minimum expenditure of crop protection compositions.

The present invention therefore also relates to a method for protection of seed and germinating plants from attack by phytopathogenic fungi, by treating the seed with an inventive composition. The invention likewise relates to the use of the inventive compositions for treatment of seed to protect the seed and the germinating plant from phytopathogenic fungi. The invention further relates to seed which has been treated with an inventive composition for protection from phytopathogenic fungi.

The control of phytopathogenic fungi which damage plants post-emergence is effected primarily by treating the soil and the above-ground parts of plants with crop protection compositions. Owing to the concerns regarding a possible influence of the crop protection compositions on the environment and the health of humans and animals, there are efforts to reduce the amount of active ingredients deployed.

One of the advantages of the present invention is that the particular systemic properties of the inventive active ingredients and compositions mean that treatment of the seed with these active ingredients and compositions not only protects the seed itself, but also the resulting plants after emergence, from phytopathogenic fungi. In this way, the immediate treatment of the crop at the time of sowing or shortly thereafter can be dispensed with.

It is likewise considered to be advantageous that the inventive active ingredients or compositions can especially also be used with transgenic seed, in which case the plant growing from this seed is capable of expressing a protein which acts against pests. By virtue of the treatment of such seed with the inventive active ingredients or compositions, merely the expression of the protein, for example an insecticidal protein, can control certain pests. Surprisingly, a further synergistic effect can be observed in this case, which additionally increases the effectiveness for protection against attack by pests.

The inventive compositions are suitable for protecting seed of any plant variety which is used in agriculture, in greenhouses, in forests or in horticulture and viticulture. In particular, this is the seed of cereals (such as wheat, barley, rye, triticale, sorghum/millet and oats), maize, cotton, soya beans, rice, potatoes, sunflower, bean, coffee, beet (for example sugar beet and fodder beet), peanut, oilseed rape, poppy, olive, coconut, cocoa, sugar cane, tobacco, vegetables (such as tomato, cucumbers, onions and lettuce), turf and ornamentals (see also below). The treatment of the seed of cereals (such as wheat, barley, rye, triticale and oats), maize and rice is of particular significance.

As also described below, the treatment of transgenic seed with the inventive active ingredients or compositions is of particular significance. This relates to the seed of plants containing at least one heterologous gene. Definition and examples of suitable heterologous genes are given below.

In the context of the present invention, the inventive composition is applied to the seed alone or in a suitable formulation. Preferably, the seed is treated in a state in which it is sufficiently stable for no damage to occur in the course of treatment. In general, the seed can be treated at any time between harvest and sowing. It is customary to use seed which has been separated from the plant and freed from cobs, shells, stalks, coats, hairs or the flesh of the fruits. For example, it is possible to use seed which has been harvested, cleaned and dried down to a moisture content of less than 15 % by weight. Alternatively, it is also possible to use seed which, after drying, for example, has been treated with water and then dried again.

When treating the seed, care must generally be taken that the amount of the inventive composition applied to the seed and/or the amount of further additives is selected such that the germination of the seed is not impaired, or that the resulting plant is not damaged. This has to be borne in mind in particular in the case of active ingredients which can have phytotoxic effects at certain application rates.

The inventive compositions can be applied directly, i.e. without containing any other components and without having been diluted. In general, it is preferable to apply the compositions to the seed in the form of a suitable formulation. Suitable formulations and methods for seed treatment are known to those skilled in the art and are described, for example, in the following documents: US 4,272,417, US 4,245,432, US 4,808,430, US 5,876,739, US 2003/0176428 A1, WO 2002/080675, WO 2002/028186.

The active ingredients usable in accordance with the invention can be converted to the customary seed dressing formulations, such as solutions, emulsions, suspensions, powders, foams, slurries or other coating compositions for seed, and also ULV formulations.

These formulations are prepared in a known manner, by mixing the active ingredients with customary additives, for example customary extenders and also solvents or diluents, dyes, wetting agents, dispersants, emulsifiers, antifoams, preservatives, secondary thickeners, adhesives, gibberellins and also water.

Useful dyes which may be present in the seed dressing formulations usable in accordance with the invention are all dyes which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of active agrochemical ingredients. Preference is given to using alkyl naphthalenesulphonates, such as diisopropyl or diisobutyl naphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Usable with preference are nonionic or anionic dispersants or mixtures of nonionic or anionic dispersants. Suitable nonionic dispersants include especially ethylene oxide/propylene oxide block polymers, alkylphenol polyglycol ethers and tristryrylphenol polyglycol ether, and the phosphated or sulphated derivatives thereof. Suitable anionic dispersants are especially lignosulphonates, polyacrylic acid salts and arylsulphonate/formaldehyde condensates.

Antifoams which may be present in the seed dressing formulations usable in accordance with the invention are all foam-inhibiting substances conventionally used for the formulation of active agrochemical ingredients. Silicone antifoams and magnesium stearate can be used with preference.

Preservatives which may be present in the seed dressing formulations usable in accordance with the invention are all substances usable for such purposes in agrochemical compositions. Examples include dichlorophene and benzyl alcohol hemiformal.

Secondary thickeners which may be present in the seed dressing formulations usable in accordance with the invention are all substances usable for such purposes in agrochemical compositions. Preferred examples include cellulose derivatives, acrylic acid derivatives, xanthan, modified clays and finely divided silica.

Adhesives which may be present in the seed dressing formulations usable in accordance with the invention are all customary binders usable in seed dressing products. Preferred examples include polyvinylpyrrolidone, polyvinyl acetate, polyvinyl alcohol and tylose.

The gibberellins which may be present in the seed dressing formulations usable in accordance with the invention may preferably be gibberellins A1, A3 (= gibberellic acid), A4 and A7; particular preference is given to using gibberellic acid. The gibberellins are known (cf. R. Wegler "Chemie der Pflanzenschutz- und Schädlingsbekampfungsmittel" [Chemistry of the Crop Protection Compositions and Pesticides], vol. 2, Springer Verlag, 1970, p. 401-412).

The seed dressing formulations usable in accordance with the invention can be used, either directly or after previously having been diluted with water, for the treatment of a wide range of different seed, including the seed of transgenic plants. In this case, additional synergistic effects may also occur in interaction with the substances formed by expression.

For treatment of seed with the seed dressing formulations usable in accordance with the invention, or the preparations prepared therefrom by adding water, all mixing units usable customarily for the seed dressing are useful. Specifically, the procedure in the seed dressing is to place the seed into a mixer, to add the particular desired amount of seed dressing formulations, either as such or after prior dilution with water, and to mix everything until the formulation is distributed homogeneously on the seed. If appropriate, this is followed by a drying process.

### Mycotoxins

In addition, the inventive treatment can reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum*, *F. asiaticum*, *F. avenaceum*, *F. crookwellense*, *F. culmorum*, *F. graminearum* (*Gibberella zeae*), *F. equiseti, F. fujikoroi*, *F. musarum*, *F. oxysporum, F. proliferatum*, *F. poae*, *F. pseudograminearum*, *F. sambucinum*, *F. scirpi*, *F. semitectum*, *F. solani, F. sporotrichoides, F. langsethiae*, *F. subglutinans*, *F. tricinctum*, *F. verticillioides* etc., and also by *Aspergillus* spec., such as *A. flavus*, *A. parasiticus, A. nomius*, *A. ochraceus*, *A. clavatus*, *A. terreus, A. versicolor, Penicillium* spec., such as *P. verrucosum*, *P. viridicatum*, *P. citrinum*, *P. expansum*, *P. claviforme*, *P. roqueforti*, *Claviceps* spec., such as *C. purpurea, C. fusiformis*, *C. paspali*, *C. africana*, *Stachybotrys* spec. and others.

### Material Protection

The inventive active ingredients or compositions can also be used in the protection of materials, for protection of industrial materials against attack and destruction by harmful microorganisms, for example fungi and insects.

In addition, the inventive compounds can be used as antifouling compositions, alone or in combinations with other active ingredients.

Industrial materials in the present context are understood to mean inanimate materials which have been prepared for use in industry. For example, industrial materials which are to be protected by inventive active ingredients from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants and heat transfer fluids, more preferably wood.

The inventive active ingredients or compositions may prevent adverse effects, such as rotting, decay, discoloration, decoloration or formation of mould.

In the case of treatment of wood the compounds/compositions according to the invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds according to the invention or a composition according to the invention; this includes for example direct application, spraying, dipping, injection or any other suitable means.

In addition, the inventive compounds can be used to protect objects which come into contact with saltwater or brackish water, especially hulls, screens, nets, buildings, moorings and signalling systems, from fouling.

The inventive method for controlling unwanted fungi can also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, can be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The inventive active ingredients may prevent adverse effects, such as rotting, decay, discoloration, decoloration or formation of mould.

Microorganisms capable of degrading or altering the industrial materials include, for example, bacteria, fungi, yeasts, algae and slime organisms. The inventive active ingredients preferably act against fungi, especially moulds, wood-discoloring and wood-destroying fungi (*Ascomycetes*, *Basidiomycetes, Deuteromycetes* and *Zygomycetes*), and against slime organisms and algae. Examples include microorganisms of the following genera: *Alternaria*, such as *Alternaria tenuis*; *Aspergillus,* such as *Aspergillus niger*; *Chaetomium*, such as *Chaetomium globosum*; *Coniophora,* such as *Coniophora puetana*; *Lentinus,* such as *Lentinus tigrinus*; *Penicillium,* such as *Penicillium glaucum*; *Polyporus,* such as *Polyporus versicolor*; *Aureobasidium,* such as *Aureobasidium pullulans*; *Sclerophoma*, such as *Sclerophoma pityophila*; *Trichoderma,* such as *Trichoderma viride*; *Ophiostoma* spp., *Ceratocystis* spp., *Humicola* spp., *Petriella* spp., *Trichurus* spp., *Coriolus* spp., *Gloeophyllum* spp., *Pleurotus* spp., *Poria* spp., *Serpula* spp. and *Tyromyces* spp., *Cladosporium* spp., *Paecilomyces* spp. *Mucor* spp., *Escherichia,* such as *Escherichia coli*; *Pseudomonas,* such as *Pseudomonas aeruginosa*; *Staphylococcus,* such as *Staphylococcus aureus, Candida* spp. and *Saccharomyces* spp., such as *Saccharomyces cerevisae.*

### Application Rates and Timing

When using the inventive active ingredients as fungicides, the application rates can be varied within a relatively wide range, depending on the kind of application. The application rate of the inventive active ingredients is
- in the case of treatment of plant parts, for example leaves: from 0.1 to 10 000 g/ha, preferably from 10 to 1000 g/ha, more preferably from 10 to 800 g/ha, even more preferably from 50 to 300 g/ha (in the case of application by watering or dripping, it is even possible to reduce the application rate, especially when inert substrates such as rockwool or perlite are used);
- in the case of seed treatment: from 2 to 200 g per 100 kg of seed, preferably from 3 to 150 g per 100 kg of seed, more preferably from 2.5 to 25 g per 100 kg of seed, even more preferably from 2.5 to 12.5 g per 100 kg of seed;
- in the case of soil treatment: from 0.1 to 10 000 g/ha, preferably from 1 to 5000 g/ha.

These application rates are merely by way of example and are not limiting for the purposes of the invention.

The inventive active ingredients or compositions comprising a compound according to formula (I) can thus be used to protect plants from attack by the pathogens mentioned for a certain period of time after treatment. The period for which protection is provided extends generally for 1 to 28 days, preferably for 1 to 14 days, more preferably for 1 to 10 days, most preferably for 1 to 7 days, after the treatment of the plants with the active ingredients, or for up to 200 days after a seed treatment.

The plants listed can particularly advantageously be treated in accordance with the invention with the compounds of the general formula (I) and the inventive compositions. The preferred ranges stated above for the active ingredients or compositions also apply to the treatment of these plants. Particular emphasis is given to the treatment of plants with the compounds or compositions specifically mentioned in the present text.

### Examples

The preparation and the use of the inventive active ingredients of the formula **(I)** is illustrated by the examples which follow. However, the invention is not limited to these examples.

**General notes:** Unless stated otherwise, all chromatographic purification and separation steps are carried out on silica gel and using a solvent gradient from 0:100 ethyl acetate/cyclohexane to 100:0 ethyl acetate/cyclohexane.

### Preparation of compound (I-3)

### Step 1

### 4-(4-{5-[2,4-difluoro-6-(prop-2-yn-1-yloxy)phenyl]-4,5-dihydro-1,2-oxazol-3-yl}-1,3-thiazol-2-yl)-N-(2,5-dimethylphenyl)piperidine-1-carboxamide (I-3)

To a suspension of 4-(4-{5-[2,4-difluoro-6-(prop-2-yn-1-yloxy)phenyl]-4,5-dihydro-1,2-oxazol-3-yl}-1,3-thiazol-2-yl)piperidinium chloride (199 mg) in dichloromethane (5 ml) and triethylamine (50 mg) at room temperature were added 2-isocyanato-1,4-dimethylbenzene (70 mg) and one drop of 1,8-diazabicyclo[5.4.0]undec-7-ene (DBU). The mixture was stirred at room temperature for 16 hours, and then water was added. The aqueous phase was removed and extracted with ethyl acetate. The combined organic phases were dried over sodium sulphate and concentrated under reduced pressure. The residue was purified by chromatography. This gave 4-(4-{5-[2,4-difluoro-6-(prop-2-yn-1-yloxy)phenyl]-4,5-dihydro-1,2-oxazol-3-yl}-1,3-thiazol-2-yl)-N-(2,5-dimethylphenyl)piperidine-1-carboxamide (69 mg).

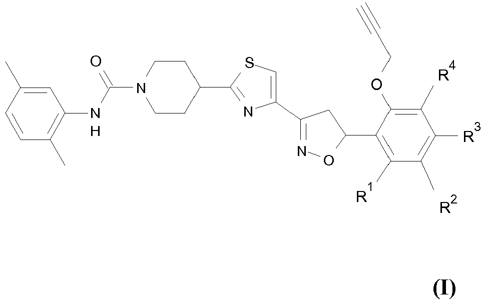

**Table 2:**

| **Ex.** | **R¹** | **R²** | **R³** | **R⁴** | **Log P** |
|---|---|---|---|---|---|
| **I-1** | fluoro | hydrogen | hydrogen | fluoro | 3.45^{[a]} |
| **I-2** | fluoro | hydrogen | hydrogen | hydrogen | 3.31^{[a]} |
| **I-3** | fluoro | fluoro | hydrogen | hydrogen | 3.45^{[a]}, 3.35^{[b]} |
| **I-4** | fluoro | hydrogen | fluoro | hydrogen | 3.47^{[a]}, 3.35^{[b]} |

Measurement of LogP values was performed according to EEC directive 79/831 Annex V.A8 by HPLC (High Performance Liquid Chromatography) on reversed phase columns with the following methods:
[a] LogP value is determined by measurement of LC-UV, in an acidic range, with 0.1% formic acid in water and acetonitrile as eluent (linear gradient from 10% acetonitrile to 95% acetonitrile).
[b] LogP value is determined by measurement of LC-UV, in a neutral range, with 0.001 molar ammonium acetate solution in water and acetonitrile as eluent (linear gradient from 10% acetonitrile to 95% acetonitrile).

Calibration was done with straight-chain alkan2-ones (with 3 to 16 carbon atoms) with known LogP values (measurement of LogP values using retention times with linear interpolation between successive alkanones). Lambda-max-values were determined using UV-spectra from 200 nm to 400 nm and the peak values of the chromatographic signals.

### NMR data of selected examples

1H-NMR data of selected examples are written in form of 1H-NMR-peak lists. To each signal peak are listed the δ-value in ppm and the signal intensity in round brackets. Between the δ-value - signal intensity pairs are semicolons as delimiters.

The peak list of an example has therefore the form:
δ₁ (intensity₁); δ₂ (intense₂);........; δᵢ (intensityᵢ);......; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for 1H spectra, we use tetramethylsilane and/or the chemical shift of the solvent used, especially in the case of spectra measured in DMSO. Therefore in NMR peak lists, tetramethylsilane peak can occur but not necessarily.

The 1H-NMR peak lists are similar to classical 1H-NMR prints and contains therefore usually all peaks, which are listed at classical NMR-interpretation.

Additionally they can show like classical 1H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-D₆ and the peak of water are shown in our 1H-NMR peak lists and have usually on average a high intensity .

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical 1H-NMR interpretation.

Further details of NMR-data description with peak lists you find in the publication "Citation of NMR Peaklist Data within Patent Applications" of the Research Disclosure Database Number 564025.

| |
|---|
| Example I-1: ¹H-NMR (400.0 MHz, d₆-DMSO): |
| δ= 8.785 (0.5); 8.019 (3.9); 8.000 (8.6); 7.439 (0.8); 7.426 (0.8); 7.416 (1.0); 7.412 (1.0); 7.403 (1.0); 7.400 (1.0); 7.389 (0.9); 7.376 (0.8); 7.135 (0.9); 7.125 (0.9); 7.110 (1.5); 7.101 (1.4); 7.087 (0.8); 7.077 (0.7); 7.049 (2.7); 7.030 (3.2); 7.000 (3.5); 6.859 (2.1); 6.841 (1.7); 6.060 (1.2); 6.037 (1.4); 6.029 (1.4); 6.007 (1.2); 4.875 (0.9); 4.869 (0.9); 4.835 (3.1); 4.829 (3.1); 4.808 (3.0); 4.802 (3.0); 4.768 (0.9); 4.762 (0.8); 4.183 (1.9); 4.149 (2.0); 4.099 (0.4); 4.086 (0.4); 3.897 (0.8); 3.864 (0.9); 3.854 (1.1); 3.851 (1.1); 3.821 (0.9); 3.650 (1.9); 3.645 (3.8); 3.639 (1.8); 3.544 (1.3); 3.522 (1.3); 3.501 (1.0); 3.479 (1.0); 3.363 (0.4); 3.355 (0.7); 3.345 (0.5); 3.322 (36.7); 3.298 (0.7); 3.288 (0.4); 3.175 (1.7); 3.161 (1.7); 3.010 (1.3); 2.981 (2.5); 2.952 (1.3); 2.675 (0.7); 2.670 (1.0); 2.666 (0.7); 2.510 (59.4); 2.506 (113.7); 2.501 (146.4); 2.497 (106.6); 2.333 (0.8); 2.328 (1.0); 2.324 (0.8); 2.234 (16.0); 2.112 (15.5); 2.068 (1.9); 2.062 (1.9); 1.710 (0.5); 1.700 (0.7); 1.679 (1.4); 1.670 (1.5); 1.648 (1.5); 1.640 (1.4); 1.619 (0.6); 1.609 (0.5); 0.008 (0.7); 0.000 (16.3); - 0.008 (0.6) |
| Example I-2: ¹H-NMR (400.0 MHz, d₆-DMSO): |
| δ= 8.019 (3.0); 7.969 (7.9); 7.435 (0.7); 7.418 (0.9); 7.414 (1.5); 7.397 (1.5); 7.393 (1.0); 7.376 (0.8); 7.048 (2.0); 7.029 (2.4); 7.012 (2.3); 7.001 (2.8); 6.991 (2.1); 6.913 (1.1); 6.892 (1.2); 6.888 (1.3); 6.865 (1.2); 6.859 (1.6); 6.840 (1.2); 6.073 (1.0); 6.050 (1.2); 6.042 (1.1); 6.020 (1.0); 4.855 (3.1); 4.849 (5.2); 4.844 (3.0); 4.183 (1.4); 4.149 (1.5); 4.055 (1.2); 4.038 (3.7); 4.020 (3.7); 4.002 (1.3); 3.815 (0.6); 3.785 (0.7); 3.782 (0.7); 3.773 (0.9); 3.770 (0.9); 3.740 (0.8); 3.548 (1.7); 3.542 (3.7); 3.536 (1.7); 3.528 (1.1); 3.506 (1.1); 3.485 (0.8); 3.463 (0.8); 3.354 (0.5); 3.344 (0.4); 3.335 (0.7); 3.323 (16.6); 3.306 (0.4); 3.297 (0.5); 3.012 (1.0); 2.982 (1.9); 2.953 (1.0); 2.523 (0.9); 2.510 (14.9); 2.505 (29.7); 2.501 (38.9); 2.496 (28.0); 2.492 (13.5); 2.233 (11.7); 2.113 (12.2); |
| 2.093 (1.3); 2.067 (1.4); 1.989 (16.0); 1.701 (0.4); 1.671 (1.1); 1.647 (1.0); 1.618 (0.4); 1.397 (4.5); 1.192 (4.3); 1.174 (8.5); 1.156 (4.2); 0.000 (6.0) |
| Example I-3: ¹H-NMR (400.0 MHz, d₆-DMSO): |
| δ= 8.020 (4.0); 7.985 (7.8); 7.509 (0.7); 7.485 (1.8); 7.461 (1.8); 7.437 (0.7); 7.049 (2.6); 7.030 (3.1); 7.002 (5.0); 6.987 (1.3); 6.983 (1.2); 6.859 (2.2); 6.840 (1.8); 6.073 (1.2); 6.051 (1.4); 6.043 (1.4); 6.020 (1.2); 5.756 (1.5); 4.845 (6.6); 4.183 (2.1); 4.150 (2.2); 4.038 (0.6); 4.020 (0.6); 3.865 (1.0); 3.835 (1.1); 3.823 (1.4); 3.792 (1.2); 3.571 (1.6); 3.563 (2.1); 3.557 (4.0); 3.551 (3.1); 3.529 (1.1); 3.507 (1.1); 3.356 (0.7); 3.323 (56.3); 3.299 (0.8); 3.013 (1.4); 2.983 (2.7); 2.953 (1.4); 2.671 (0.8); 2.506 (96.3); 2.502 (116.8); 2.329 (0.8); 2.234 (15.5); 2.113 (16.0); 2.067 (2.1); 1.989 (2.4); 1.702 (0.7); 1.675 (1.6); 1.647 (1.5); 1.619 (0.5); 1.193 (0.6); 1.175 (1.2); 1.157 (0.6); 0.008 (1.9); 0.000 (36.3) |
| Example I-4: ¹H-NMR (400.0 MHz, d₆-DMSO): |
| δ= 8.019 (4.0); 7.966 (8.6); 7.049 (2.7); 7.030 (3.2); 7.002 (3.6); 6.973 (1.6); 6.960 (1.3); 6.952 (1.4); 6.944 (1.9); 6.937 (1.8); 6.933 (1.6); 6.910 (1.1); 6.905 (0.8); 6.859 (2.1); 6.841 (1.8); 6.020 (1.3); 5.997 (1.6); 5.989 (1.5); 5.967 (1.3); 5.756 (9.7); 4.888 (4.3); 4.883 (6.4); 4.878 (3.9); 4.182 (2.0); 4.148 (2.1); 4.056 (0.4); 4.038 (1.1); 4.020 (1.1); 4.002 (0.4); 3.807 (0.9); 3.776 (1.0); 3.765 (1.3); 3.734 (1.1); 3.576 (1.9); 3.570 (4.0); 3.565 (1.8); 3.512 (1.4); 3.491 (1.4); 3.470 (1.1); 3.448 (1.1); 3.351 (1.0); 3.325 (112.2); 3.294 (0.9); 3.011 (1.4); 2.981 (2.6); 2.952 (1.4); 2.675 (0.6); 2.671 (0.8); 2.666 (0.6); 2.506 (93.1); 2.502 (116.1); 2.497 (83.7); 2.333 (0.6); 2.329 (0.8); 2.324 (0.6); 2.234 (16.0); 2.207 (0.4); 2.112 (15.8); 2.064 (2.0); 1.989 (4.9); 1.697 (0.6); 1.669 (1.5); 1.645 (1.4); 1.617 (0.6); 1.250 (0.4); 1.193 (1.3); 1.175 (2.6); 1.157 (1.3); 0.000 (1.5) |

### Use Examples

### Example A

### Phytophthora test (tomatoes) / preventive

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. After the spray coating has dried on, the plants are inoculated with an aqueous spore suspension of ***Phytophthora infestans.*** The plants are then placed in an incubation cabinet at approximately 20 °C and a relative atmospheric humidity of 100%.

The test is evaluated 3 days after the inoculation. 0% means an efficacy which corresponds to that of the untreated control, while an efficacy of 100% means that no disease is observed.

Under these conditions, at a dose of 1 ppm the following examples have shown biological efficacy higher than or equal to 70%: I-1, I-2, I-3 and I-4.

### Example B

### Plasmopara test (grapevines) / preventive

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. After the spray coating has dried on, the plants are inoculated with an aqueous spore suspension of ***Plasmopara viticola*** and then remain for 1 day in an incubation cabinet at approximately 20 °C and a relative atmospheric humidity of 100%. The plant is subsequently placed for 4 days in a greenhouse at approximately 21 °C and a relative atmospheric humidity of approximately 90%. The plants are then misted and placed for 1 day in an incubation cabinet.

The test is evaluated 6 days after the inoculation. 0% means an efficacy which corresponds to that of the untreated control, while an efficacy of 100% means that no disease is observed.

Under these conditions, at a dose of 1 ppm the following examples have shown biological efficacy higher than or equal to 70%: I-1, I-2, I-3 and I-4.

## Claims

1. Compounds of the formula **(I)** 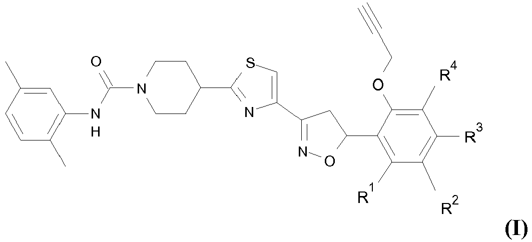 in which the radicals are defined as follows:
R¹, R², R³ and R⁴ are independently of each other H or fluorine, wherein at least one of these substituents is a fluorine atom.

2. Compound of the formula (I) according to claim 1 wherein
R¹ is fluoro;
R² is hydrogen;
R³ is hydrogen;
R⁴ is fluoro.

3. Compound of the formula (I) according to claim 1 wherein
R¹ is fluoro;
R² is hydrogen;
R³ is hydrogen;
R⁴ is hydrogen.

4. Compound of the formula (I) according to claim 1 wherein
R¹ is fluoro;
R² is fluoro;
R³ is hydrogen;
R⁴ is hydrogen.

5. Compound of the formula (I) according to claim 1 wherein
R¹ is fluoro;
R² is hydrogen;
R³ is fluoro;
R⁴ is hydrogen.

6. Method for controlling phytopathogenic harmful fungi, **characterized in that** compounds of the formula (I) according to any of Claims 1 to 5 are applied to the phytopathogenic harmful fungi and/or their habitat.

7. Composition for controlling phytopathogenic harmful fungi, **characterized by** a content of at least one compound of the formula (I) according to any of Claims 1 to 5, in addition to extenders and/or surfactants.

8. Use of compounds of the formula (I) according to any of Claims 1 to 5 for controlling phytopathogenic harmful fungi.

9. Process for producing compositions for controlling phytopathogenic harmful fungi, **characterized in that** compounds of the formula (I) according to any of Claims 1 to 5 are mixed with extenders and/or surfactants.

10. Use of compounds of the formula (I) according to any of Claims 1 to 5 for treatment of transgenic plants.

## Patentansprüche

1. Verbindungen der Formel (**I**) 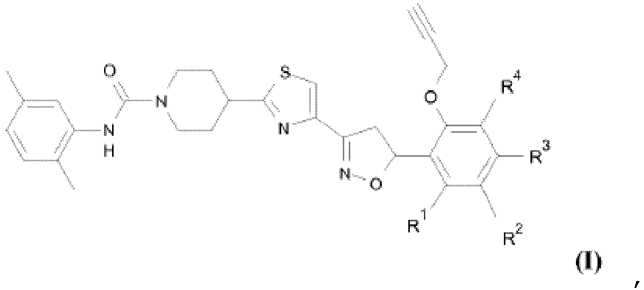 worin die Reste wie folgt definiert sind:
R¹, R², R³ und R⁴ bedeuten unabhängig voneinander H oder Fluor, wobei mindestens einer dieser Substituenten ein Fluoratom ist.

2. Verbindung der Formel (I) nach Anspruch 1, wobei
R¹ Fluor bedeutet;
R² Wasserstoff bedeutet;
R³ Wasserstoff bedeutet;
R⁴ Fluor bedeutet.

3. Verbindung der Formel (I) nach Anspruch 1, wobei
R¹ Fluor bedeutet;
R² Wasserstoff bedeutet;
R³ Wasserstoff bedeutet;
R⁴ Wasserstoff bedeutet.

4. Verbindung der Formel (I) nach Anspruch 1, wobei
R¹ Fluor bedeutet;
R² Fluor bedeutet;
R³ Wasserstoff bedeutet;
R⁴ Wasserstoff bedeutet.

5. Verbindung der Formel (I) nach Anspruch 1, wobei
R¹ Fluor bedeutet;
R² Wasserstoff bedeutet;
R³ Fluor bedeutet;
R⁴ Wasserstoff bedeutet.

6. Verfahren zum Bekämpfen von phytopathogenen Schadpilzen, **dadurch gekennzeichnet, dass** man Verbindungen der Formel (I) nach einem der Ansprüche 1 bis 5 auf die phytopathogenen Schadpilze und/oder ihren Lebensraum ausbringt.

7. Zusammensetzung zur Bekämpfung von phytopathogenen Schadpilzen, **dadurch gekennzeichnet, dass** sie mindestens eine Verbindung der Formel (I) nach einem der Ansprüche 1 bis 5 sowie Streckmittel und/oder Tenside enthält.

8. Verwendung von Verbindungen der Formel (I) nach einem der Ansprüche 1 bis 5 zum Bekämpfen von phytopathogenen Schadpilzen.

9. Verfahren zur Herstellung von Zusammensetzungen zum Bekämpfen von phytopathogenen Schadpilzen, **dadurch gekennzeichnet, dass** man Verbindungen der Formel (I) nach einem der Ansprüche 1 bis 5 mit Streckmitteln und/oder Tensiden mischt.

10. Verwendung von Verbindungen der Formel (I) nach einem der Ansprüche 1 bis 5 für die Behandlung von transgenen Pflanzen.

## Revendications

1. Composés de formule (I) 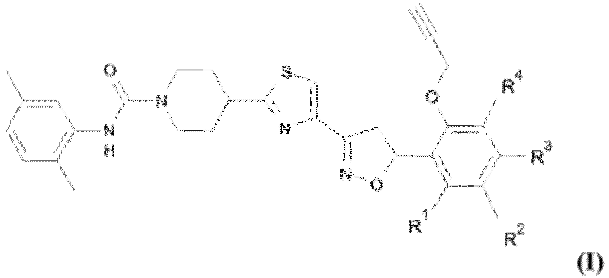 dans lesquels les radicaux sont définis tel que ci-après :
R¹, R², R³ et R⁴ sont, indépendamment les uns des autres, H ou fluor, où au moins l'un parmi ces substituants est un atome de fluor.

2. Composé de formule (I) selon la revendication 1, dans lequel
R¹ est fluoro ;
R² est hydrogène ;
R³ est hydrogène ;
R⁴ est fluoro.

3. Composé de formule (I) selon la revendication 1, dans lequel
R¹ est fluoro ;
R² est hydrogène ;
R³ est hydrogène ;
R⁴ est hydrogène.

4. Composé de formule (I) selon la revendication 1, dans lequel
R¹ est fluoro ;
R² est fluoro ;
R³ est hydrogène ;
R⁴ est hydrogène.

5. Composé de formule (I) selon la revendication 1, dans lequel
R¹ est fluoro ;
R² est hydrogène ;
R³ est fluoro ;
R⁴ est hydrogène.

6. Méthode de contrôle de champignons phytopathogènes néfastes, **caractérisée en ce que** des composés de formule (I) selon l'une quelconque des revendications 1 à 5 sont appliqués aux champignons phytopathogènes néfastes et/ou à leur habitat.

7. Composition de contrôle de champignons phytopathogènes néfastes, **caractérisée par** une teneur en au moins un composé de formule (I) selon l'une quelconque des revendications 1 à 5, outre des matières de charge et/ou des agents tensioactifs.

8. Utilisation de composés de formule (I) selon l'une quelconque des revendications 1 à 5, pour le contrôle de champignons phytopathogènes néfastes.

9. Procédé de production de compositions destinées au contrôle de champignons phytopathogènes néfastes, **caractérisé en ce que** des composés de formule (I) selon l'une quelconque des revendications 1 à 5 sont mélangés avec des matières de charge et/ou des agents tensioactifs.

10. Utilisation de composés de formule (I) selon l'une quelconque des revendications 1 à 5, pour le traitement de plantes transgéniques.
